# EUROPEAN PATENT APPLICATION

(11) **EP 4 708 214 A2**
(43) Date of publication of application: **11.03.2026**
(21) Application number: 25226102.9
(22) Date of filing: 04.01.2018
(51) Int. Cl.: G06V 10/25

(54) **IDENTIFICATION OF A PREDEFINED OBJECT IN A SET OF IMAGES FROM A MEDICAL IMAGE SCANNER DURING A SURGICAL PROCEDURE**

(62) Divisional of application: 18150376.4
(71) Applicant: Augmedics Inc., Arlington Heights, IL 60005 (US)
(72) Inventor: Siemionow, Krzysztof B., Arlington Heights, 60005 (US); Luciano, Cristian J., Arlington Heights, 60005 (US); Trzmiel, Michal, Arlington Heights, 60005 (US); Fularz, Michal, Arlington Heights, 60005 (US); Mejia, Isaac, Arlington Heights, 60005 (US)
(74) Representative: Kancelaria Eupatent.pl Sp. z o.o.

(57) **Abstract**

A computer-implemented system, comprising: at least one nontransitory processor-readable storage medium that stores at least one of processor-executable instructions or data; and at least one processor communicably coupled to at least one nontransitory processor-readable storage medium, wherein at least one processor: reads (811) the set of images, each comprising an image of a registration grid (181) comprising an array of markers (181B); detects (812) the markers on each of the images by using a marker detection convolutional neural network (CNN); filters (821) the detection results to remove false positives; and determines (831) the 3D location and 3D orientation of the registration grid (181) based on the detected markers (181B).

## Description

### TECHNICAL FIELD

The present disclosure relates to computer assisted surgical navigation systems, in particular to a system and method for operative planning, image acquisition, patient registration, calibration, and execution of a medical procedure using an augmented reality image display. In particular, it relates to the identification and tracking (determination of the 3D position and orientation) of a predefined object in a set of images from a medical image scanner during a surgical procedure.

### BACKGROUND

Image guided or computer assisted surgery is a surgical procedure where the surgeon uses trackable surgical instruments combined with preoperative or intraoperative images in order to provide guidance for the procedure. Image guided surgery can utilize images acquired intraoperatively, provided for example from computer tomography (CT) scanners.

The first critical step in every computer assisted surgical navigation consists of the registration of an intraoperative scan of the patient to a known coordinate system of reference (patient's anatomy registration). In order to accurately perform this registration process, it is crucial to identify and determine the 3D transformation (position and orientation) of a fully-known predefined object, such as a registration grid, in the 3D volumetric dataset created from a series of 2D images generated by an intraoperative scanner.

A European patent EP1599148 discloses a combined computed tomography (CT) imageable fiducial locator head, an integral bone screw, and an integral divot that receives a positioning wand of an image-guided surgical (IGS) workstation. It requires replacing spheres with divots during the registration process. Spheres are used on the first stage that involves patient scanning. The spheres are directly attached to the patient's body, which requires drilling many holes in the body, and therefore can be dangerous to the patient. Later, the user has to unscrew spheres and attach divots. Then, the user has to manually touch the tool ("wand") tip to all divots to perform patient registration (there is also a reference divot placed somewhere on the table). This approach is not fully automatic, it requires a significant amount of preparations (replacing spheres) and user input (touching divots). The accuracy of this method depends on divots positioning and surgeon skills in placing the tooltip inside of the spheres.

A US patent US7359746 discloses an image-guided interventional system that enables registration of a patient, preferably automatically and without an explicit patient registration step, to newly acquired images as the patient is moved out of the medical scanner. The system includes a calibration step, involving the use of a base structure (grid) that contains a plurality of both retro-reflective markers and radiopaque fiducials. The retro-reflective markers are visible by the optical tracker but not visible by the medical scanner. On the other hand, the radiopaque fiducials are visible by the scanner but not by the optical tracker. Therefore, knowing the relationship between the fiducials and the markers, the system requires to determine the positions of the fiducials in the coordinate system of the optical tracker by computing a transformation matrix that relates or maps the physical location of the fiducials to the positions of the fiducials in the images of the scanner. However, no particular and detailed procedure has been proposed to identify and locate these fiducials in the images obtained by the medical scanner. That system presents the following drawbacks: 1) the grid must contain both markers and fiducials, so it is quite complex to design and expensive to manufacture, 2) it needs to properly identify and locate two different types of elements (markers and fiducials) in two different image modalities (stereo images and volume respectively), and 3) the computational approach is cumbersome, as they indirectly determine the position and orientation of all radiopaque fiducials in the coordinate system of the optical tracker by tracking the retro-reflective markers, identifying the fiducials from the images, and computing the relationship between the markers and the fiducials.

A US patent US7561733 discloses a method and device for reciprocally assigning a spatial position of a patient and/or a part of a patient's body and an acquired, stored patient data set includes producing at least two video image recordings of the patient and/or part of the body from different recording positions via at least one video image source, wherein a three-dimensional position and/or orientation of the video image source relative to a reference coordinate system is known or can be determined. The tracking system can ascertain the three-dimensional spatial position of the video image source, while the video image source obtain at least two video recordings. Recordings are produced while the video image source is located within the detection range of the tracking system. The video image source is responsible for characteristic points identification. The stored patient data sets are later matched to a point set of the assigned spatial positions. The use of a stereo camera system and a tracker at the same time introduces high demand on the hardware and software necessary to operate the system. In addition, if the patient or part of the patient's body is shifted or its shape is changed (e.g., brain shift), then re-assignment and/or re-registration processes must be performed once again.

A US patent US8010177 discloses a system to allow registration of a patient to image space. The registration process can eliminate manual steps of image-to-patient registration and image-to-image registration. The system uses fiducial markers that are fixedly interconnected with a patient and then identified on the images. However, no particular and detailed procedure has been proposed to identify and track these fiducial markers.

There is a need for an efficient method to identify and determine the 3D transformation (of position and orientation) of a predefined object (such as a registration grid with fiducial markers) in a series of images from an intraoperative scanner, to enable proper identification of the patient anatomy during the surgical procedure. The present disclosure proposes a much simpler and more accurate solution based on a neural network algorithm for identification, tracking, and registration.

### SUMMARY

There is disclosed a computer-implemented system, comprising: at least one nontransitory processor-readable storage medium that stores at least one of processor-executable instructions or data; and at least one processor communicably coupled to at least one nontransitory processor-readable storage medium. The at least one processor: reads the set of images, each comprising an image of a registration grid comprising an array of markers; detects the markers on each of the images by using a marker detection convolutional neural network; filters the detection results to remove false positives; and determines the 3D location and 3D orientation of the registration grid based on the detected markers.

At least one processor further: receives marker detection learning data comprising a plurality of batches of labeled anatomical image sets, each image set comprising image data representative of an anatomical structure, and each image set including at least one marker; trains a fully convolutional neural network model (marker detection CNN) to detect markers on the images using the received marker detection learning data; and stores the trained marker detection CNN model in at least one nontransitory processor-readable storage medium of the machine learning system.

At least one processor further: receives denoising learning data comprising a plurality of batches of high quality medical imagery and low quality medical imagery; trains a fully convolutional neural network model (denoising CNN) to denoise an image utilizing the received denoising learning data; and stores the trained denoising CNN model in at least one nontransitory processor-readable storage medium of the machine learning system.

At least one processor further operates the trained marker detection CNN to process a set of input anatomical images to detect markers.

At least one processor further operates the trained denoising CNN to process a set of input anatomical images to generate a set of output denoised anatomical images.

The set of input anatomical images for the trained denoising CNN comprises the low quality anatomical images.

The set of input anatomical images for the trained marker detection CNN comprises the set of output denoised anatomical images of the denoising CNN or raw data scan..

The low quality anatomical images are low-dose computed tomography or low-power magnetic resonance images and wherein the high quality anatomical images are high-dose computed tomography or high power magnetic resonance images, respectively.

There is also disclosed a method for identification of transformation of a predefined object in a set of images from medical image scanner, the method comprising the steps of: reading the set of images, each comprising an image of a registration grid comprising an array of markers; detecting the markers on each of the images by using a marker detection convolutional neural network; filtering the detection results to remove false positives; and determining the 3D location and 3D orientation of the registration grid based on the detected markers.

### BRIEF DESCRIPTION OF DRAWINGS

The present invention is shown by means of example embodiments on a drawing, wherein:
Fig. 1A shows an example of a registration grid;
Figs. 1B-1F show examples of input and output data;
Figs. 1G, 1H show other possible examples of a registration grid;
Figs. 2A-2D show low quality scans and corresponding denoised images;
Fig. 3 shows a denoising CNN architecture;
Fig. 4 shows a marker detection CNN architecture;
Fig. 5 shows a flowchart of a marker detection CNN training process;
Fig. 6 shows flowchart of an inference process for denoising CNN;
Fig. 7 shows a flowchart of an inference process for the marker detection CNN;
Fig. 8 shows an overview of the method presented herein;

### DETAILED DESCRIPTION

The present disclosure relates to processing images of a patient anatomy, such as a bony structure (e.g., spine, skull, pelvis, long bones, joints, etc.). The foregoing description will present examples related mostly to a spine, but a skilled person will realize how to adapt the embodiments to be applicable to the other anatomical structures (e.g., blood vessels, solid organs like the heart or kidney, and nerves) as well.

During the process of setting up a computer-assisted surgical navigation system, it is needed to find a relationship between the patient internal anatomy (which is not exposed during minimally invasive surgery), and the tracking system. The method described in this patent involves scanning the patient anatomy along with a predefined object that is visible by both the scanner and the tracker. For example, a computer tomography (CT) scanner can be used.

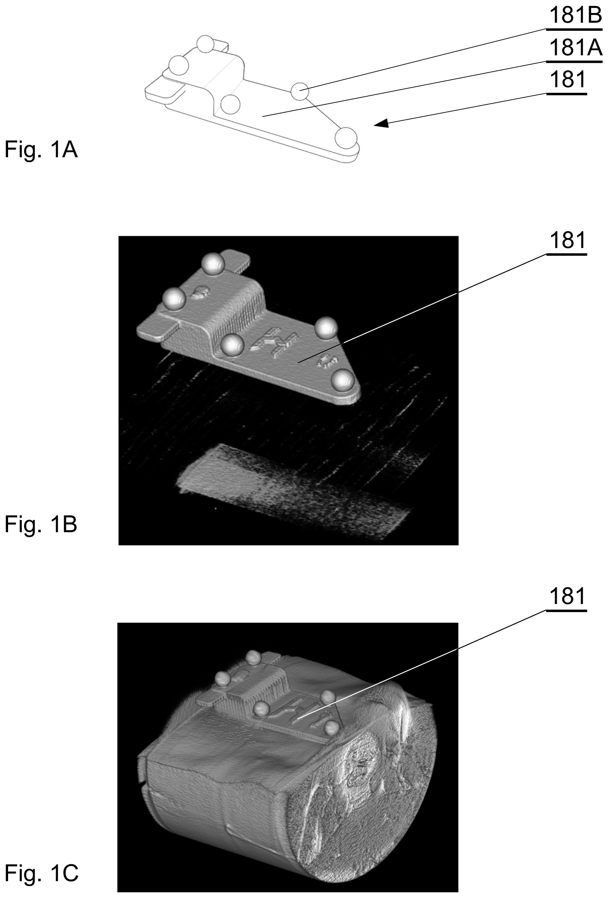

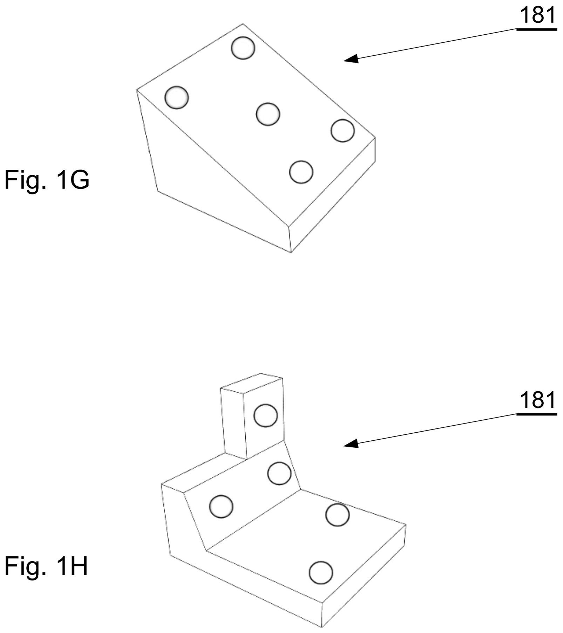

That predefined object can be a registration grid 181, such as shown in Fig. 1A, 1G or 1H. The registration grid may have a base 181A and an array of fiducial markers 181B that are registrable by the medical image scanner and tracker. For enhanced registration results, the registration grid 181 compromises of five or more reflective markers. Three of them are used to define a frame of reference for 3D orientation and position, and the remaining two or more are used to increase the tracking accuracy. The registration grid 181 can be attached to the patient for example by adhesive, such that it stays in position during the process of scanning.

To find the relationship between the patient anatomy and the tracking system, a process called registration is performed. Two images are used in the registration procedure.

The first image is a volume comprising the image of the registration grid, as shown in Fig. 1B. The second image is a volume created based on a set of scans from an intraoperative scanner and comprises the image of the registration grid and the anatomical structure, such as the spine, as shown in Fig. 1C.

Both volumes have similar parts. The aim of the method is to find the transformation between them, meaning how to rotate and move the first volume (the registration grid) to match the corresponding part on the second volume (the anatomical structure with the registration grid). In order to achieve this, an initial estimation (prealignment of the aforementioned transformation (rotation, translation, and scale) is performed.

In general terms, the method finds characteristic features of the object (grid) in both volumes and based on these features, it finds the homogeneous transformation (defining translation and orientation) between them. The characteristic features of the object can be spheres (fiducial markers) on the registration grid.

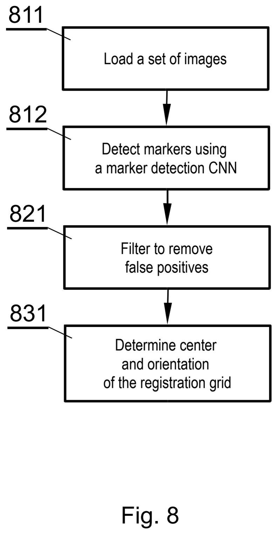

The whole method can be separated into three main processes, as described in details in fig. 8:
1) Loading set of images and finding registration grid markers,
2) Filtering the results to remove false positives,
3) Determining the grid position and orientation based on the centers of found markers.

### 1) Loading set of images and finding grid markers

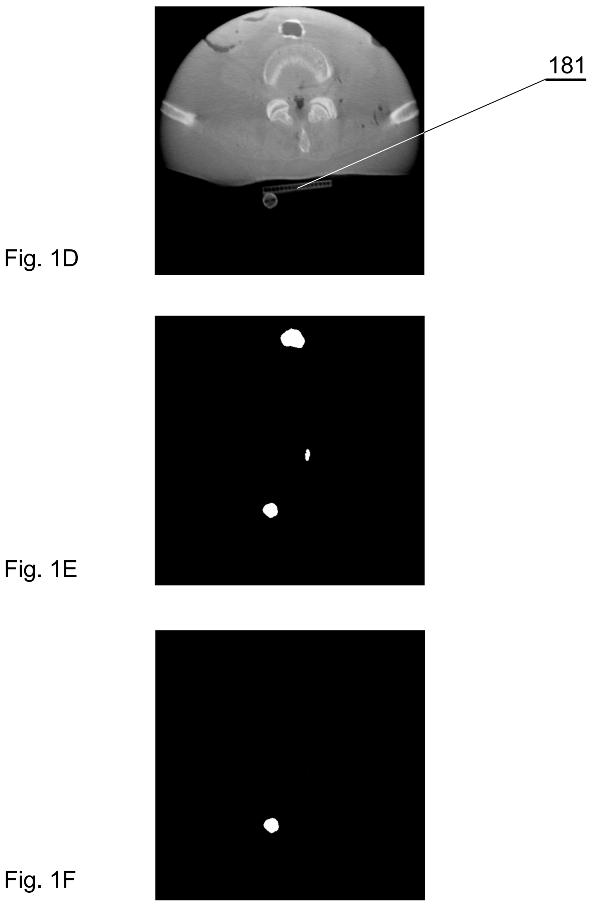

In step 811, a set of DICOM images, such as shown in Fig. 1D are loaded from a medical image scanner.

In step 812, each image (slice) is the input of a marker detection Convolutional Neural Network (CNN) with skip connections. The trained CNN individually processes each image (slice) and returns a binary image with pixels that correspond to the markers set to 1, otherwise to 0. For example, Fig. 1E shows a result of processing of the image of Fig. 1D by neural network model.

### 2) Filtering the results to remove false positives

In step 821, the images are filtered, as the marker finding routine may give some false positives (i.e., some pixels are marked as belonging to markers, while they should not be). For example, Fig. 1E shows both a blood vessel (top) and a false positive element (middle). Only the white object at the bottom is the true positive result. The false positive artifacts shall be removed.

This step is based on the assumption that the markers have a known size and shape. It can be based on a labeling process known as a connected-component labeling or any improvement thereof. The implemented method takes advantage of the nature of the marker: they occupy only small part of the volume. The labeling process connects voxels that are neighbors and creates blobs. Then it filters the list of blobs based on their size (number of voxels in each marker).

This step is performed on a whole set of images at once to take advantage of voxel connectivity (3D neighbourhood). It performs labeling of neighbouring voxels so they are grouped into blobs, which are filtered based on the amount of voxels that represent markers. For each of the resulting blobs (e.g., five in the case of the grid shown in Fig. 1B, but the exact amount can be changed in another grid), the method calculates their centers. An example of filtered image of Fig. 1E is shown in Fig. 1F.

### 3) Determining registration grid position and orientation

In step 831, the 3D location of the center of the grid and its 3D orientation are computed. This is based on the locations of markers provided from the previous step and prior knowledge of grid dimensions (given by the CAD software that was used to design the grid). This process first sorts the markers found in the scan volume, so they are aligned in the same way as the markers in the original grid, then it calculates two vectors in each grid. These vectors are used to create homogeneous transformation matrices that describe the position and orientation of the registration grid in the volume. The result of the multiplication of those matrices is the required transformation.

The main problem with finding the markers on images is that their position is unknown. It can be different for each patient and each dataset. Moreover, the design of the grid base, marker layout, and even number of markers can also differ, and might change depending on the registration grid used. Each dataset received from scanner is noisy and the amount of that noise is related to settings of the intraoperative CT scan, and interference with objects (such as surgical table, instruments, and equipment) in the operating room environment. The method responsible for finding all markers on scans should consider all of the problems mentioned above. It should be immune to the markers position and be able to find all of them regardless of their number, layout, and amount of noise in the scan dataset.

The markers are found by the marker detection neural network as described below. It is a convolutional neural network (CNN) with skip connections between some layers. That allows the network to combine detected object features from different stages of the prediction. A single binary mask is used as an output. Each pixel value of the network output corresponds to the probability of the marker occurrence on a specific position of the image. Pixel value range is 0 - 1, where 1 is interpreted as 100% certainty that this pixel is part of the marker, and 0 corresponds to 0% certainty.

The most important feature of convolutional neural networks is that they are invariant to scale, rotation, and translation. This means that the algorithm is able to find the markers on each image regardless of the marker's location, orientation and size. This invariance is achieved thanks to the way how the convolutional network works. It tries to find different features of the objects (like round shapes, straight lines, corners, etc.), and then, based on their relative location, it decides if this specific part of the image represents the markers or not, in turn labeling it with a probability value (from 0 to 1). All feature detectors are applied on every possible location on the image, so object's attributes (for example round contours) are found regardless of the object's location on the image.

In order to achieve acceptable results, regardless of the grid design or scan quality, a large dataset of training samples is required. Training the network requires a dataset of several (preferably, a few hundred or a few thousand) intraoperative scans (for example, of a spine phantom). This dataset can be split into two separate subsets. The bigger one can be used for network training and the smaller one only for testing and validation. After training, the network is ready to efficiently work on any image data and thus, its prediction time is very small. The outcomes of subsequent detections, with small adjustments made by the user, can be used as training data for new version of the neural network model to further improve the accuracy of the method.

The most innovative part of the approach for registration presented herein is the fact that the prealignment process is fully automatic and invariant to grid type, as well as 3D position and orientation, and it can even be used with different anatomical parts of the patient. It's based on an artificial intelligence algorithm, which is constantly learning and improving its performance in terms of both accuracy and processing time.

The possibility of using artificially-created objects (e.g., 3D printed models of the anatomical structure) for training the neural network makes training and gathering of the data significantly easier. The main challenge of neural network training is to obtain a high variety of learning samples. With 3D printed spine models for example, an arbitrary number as scans can be performed, to train the network on different scenarios, and the neural network knowledge will still be applicable to recognize markers on real patient data. During training different conditions can be simulated, such as rotations, translations, zoom, brightness changes, etc. Similar steps can be repeated with other anatomical structures such as the heart, kidney, or aorta. In addition, a denoising network can be used to create the versatile spherical marker detector.

Another advantage of the approach presented herein is the fact that the method operates directly on raw pixel values from the intraoperative scanner, without any significant preprocessing. That way this approach is highly robust.

The following are novel advantages of the method presented herein, taken individually or in combination with each other:
- it is heavily based on a deep learning algorithm (convolutional neural networks) that is fully automatic and it is constantly learning and improving over time;
- Registration grid segmentation is invariant to scale, rotation, and translation of the markers. Different designs of the grid and marker layouts can be used depending on the clinical setting;
- the method can be used with various anatomical parts of the body. Neural network works directly on pixel values from intraoperative scanner;
- real patient data and artificially-created objects (3D anatomical part models) are used to create large training dataset with huge variety of learning samples;
- the process is fully automatic and does not require any human intervention.

The images input to the marker detection CNN may be first subject to pre-processing of lower quality images to improve their quality, such as denoising. For example, the lower quality images may be low dose computed tomography (LDCT) images or magnetic resonance images captured with a relatively low power scanner. The foregoing description will present examples related to computed tomography (CT) images, but a skilled person will realize how to adapt the embodiments to be applicable to other image types, such as magnetic resonance images.

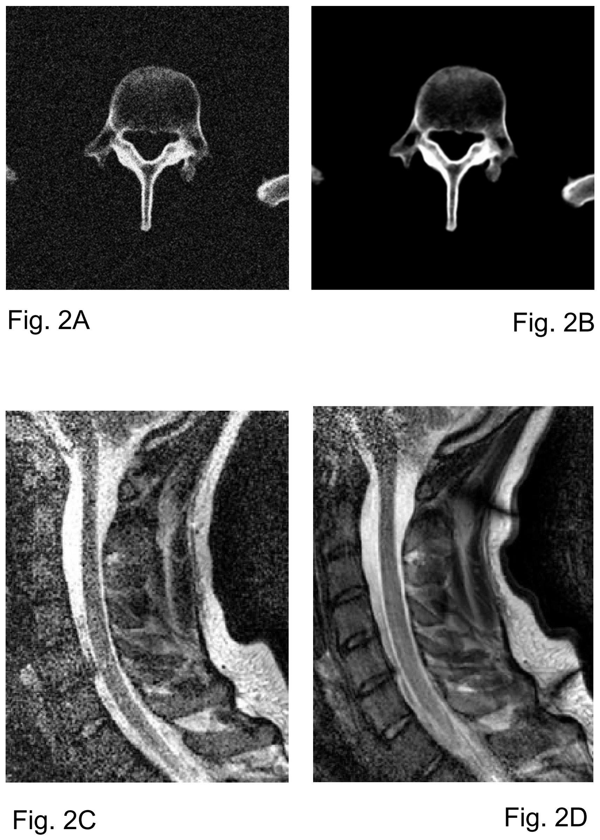

Figs. 2A and 2B show an enlarged view of a CT scan, wherein Fig. 2A is an image with a high noise level, such as a low dose (LDCT) image, and Fig. 2B is an image with a low noise level, such as a high dose (HDCT) image, or a LDCT image denoised according to the method presented herein.

Fig. 2C shows a low strength magnetic resonance scan of a cervical portion of the spine and Fig. 2D shows a high strength magnetic resonance scan of the same cervical portion (wherein Fig. 2D is also the type of image that is expected to be obtained by performing denoising of the image of Fig. 2C).

Therefore, in the present invention, a low-dose medical imagery (such as shown in Fig. 2A, 2C) is pre-processed to improve its quality to the quality level of a high-dose or high quality medical imagery (such as shown in Fig. 2B, 2D), without the need to expose the patient to a high dose of radiation.

For the purposes of this disclosure, the LDCT image is understood as an image which is taken with an effective dose of X-ray radiation lower than the effective dose for the HDCT image, such that the lower dose of X-ray radiation causes appearance of higher amount of noise on the LDCT image than the HDCT image. LDCT images are commonly captured during intra-operative scans to limit the exposure of the patient to X-ray radiation.

As seen by comparing Figs. 2A and 2B, the LDCT image is quite noisy and is difficult to be automatically processed by a computer to identify the components of the anatomical structure.

The system and method disclosed below use a deep learning based (neural network) approach. In order for any neural network to work, it must first be trained. The learning process is supervised ( i.e., the network is provided with a set of input samples and a set of corresponding desired output samples). The network learns the relations that enable it to extract the output sample from the input sample. Given enough training examples, the expected results can be obtained.

In the presented method, a set of samples are generated first, wherein LDCT images and HDCT images of the same object (such as an artificial 3D printed model of the lumbar spine) are captured using the computer tomography device. Next, the LDCT images are used as input and their corresponding HDCT images are used as desired output to learn the neutral network to denoise the images. Since the CT scanner noise is not totally random (there are some components that are characteristic for certain devices or types of scanners), the network learns which noise component is added to the LDCT images, recognizes it as noise, and it is able to eliminate it in the following operation when a new LDCT image is provided as an input to the network.

By denoising the LDCT images, the presented system and method may be used for intra-operative tasks, to provide high segmentation quality for images obtained from intra-operative scanners on low radiation dose setting.

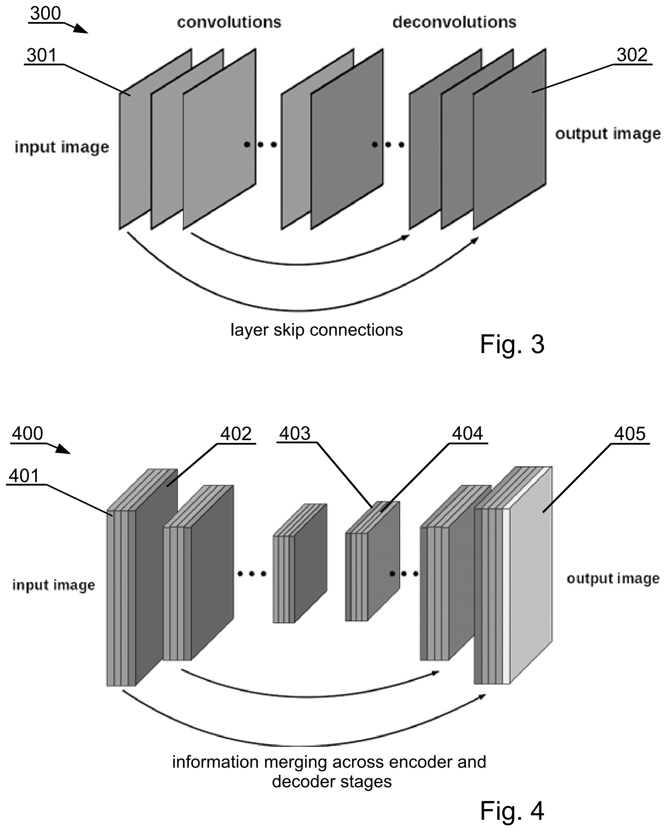

Fig. 3 shows a convolutional neural network (CNN) architecture 300, hereinafter called the denoising CNN, which is utilized in the present method for denoising. The network comprises convolution layers 301 (with ReLU activation attached) and deconvolution layers 302 (with ReLU activation attached). The use of a neural network in place of standard de-noising techniques provides improved noise removal capabilities. Moreover, since machine learning is involved, the network can be fine-tuned to specific noise characteristics of the imaging device to further improve the performance.

Fig. 4 shows a marker detection CNN architecture 400. The network performs pixel-wise class assignment using an encoder-decoder architecture, using as an input the raw images or pre-processed (e.g. denoised) images. The left side of the network is a contracting path, which includes convolution layers 401 and pooling layers 402, and the right side is an expanding path, which includes upsampling or transpose convolution layers 403 and convolutional layers 404 and the output layer 405.

One or more images can be presented to the input layer of the network to learn reasoning from a single slice image, or from a series of images fused to form a local volume representation. The convolution layers 401, as well as the upsampling or deconvolution layers 403, can be of standard, dilated, or a combination thereof, with ReLU or leaky ReLU activation attached.

The output layer 405 denotes the densely connected layer with one or more hidden layer and a softmax or sigmoid stage connected as the output.

The encoding-decoding flow is supplemented with additional skipping connections of layers with corresponding sizes (resolutions), which improves performance through information merging. It enables either the use of max-pooling indices from the corresponding encoder stage to downsample, or learning the deconvolution filters to upsample.

The final layer for marker detection recognizes two classes: the marker as foreground and the rest of the data as background.

Both architectures CNN 300 and CNN 400 are general, in the sense that adopting them to images of different size is possible by adjusting the size (resolution) of the layers. The number of layers and number of filters within a layer is also subject to change, depending on the requirements of the application. Deeper networks typically give results of better quality. However, there is a point at which increasing the number of layers/filters does not result in significant improvement, but significantly increases the computation time and decreases the network's capability to generalize, making such a large network impractical.

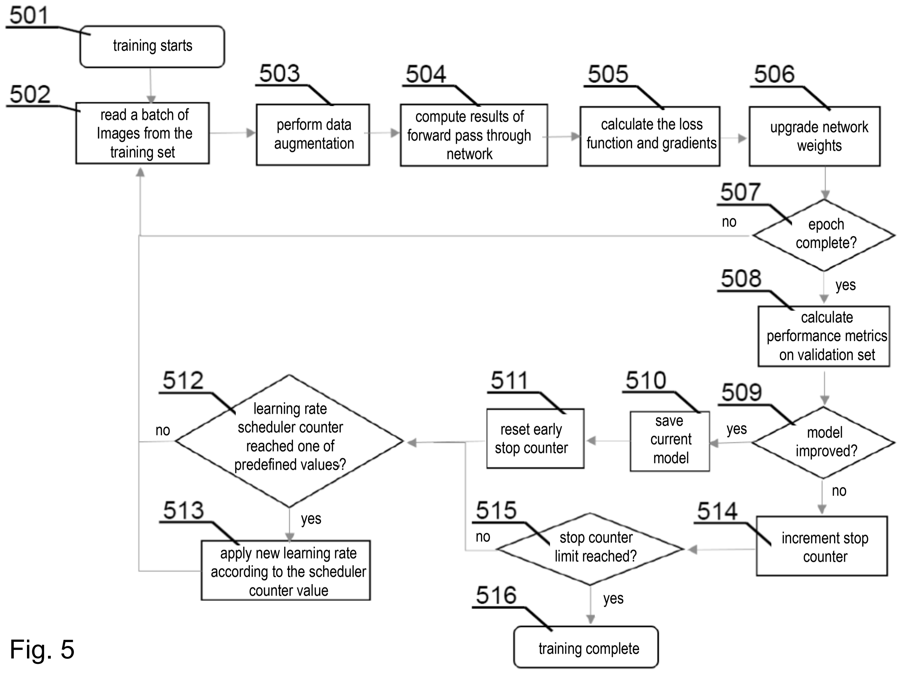

Fig. 5 shows a flowchart of a training process, which can be used to train the marker detection CNN 400.

The objective of the training for the marker detection CNN 400 is to fine-tune the parameters of the marker detection CNN 400 such that the network is able to recognize marker position on the input image (such as in Fig. 1D) to obtain an output image (such as shown in Fig. 1E or 1F).

The training database may be split into a training set used to train the model, a validation set used to quantify the quality of the model, and a test set. The training starts at 501. At 502, batches of training images are read from the training set, one batch at a time. Scans obtained from the medical image scanner represent input, and images with manually indicated markers represent output.

At 503 the images can be augmented. Data augmentation is performed on these images to make the training set more diverse. The input/output image pair is subjected to the same combination of transformations from the following set: rotation, scaling, movement, horizontal flip, additive noise of Gaussian and/or Poisson distribution and Gaussian blur, etc.

At 504, the images and generated augmented images are then passed through the layers of the CNN in a standard forward pass. The forward pass returns the results, which are then used to calculate at 505 the value of the loss function - the difference between the desired output and the actual, computed output. This difference can be expressed using a similarity metric (e.g., mean squared error, mean average error, categorical cross-entropy or another metric).

At 506, weights are updated as per the specified optimizer and optimizer learning rate. The loss may be calculated using any loss function (e.g., per-pixel cross-entropy), and the learning rate value may be updated using optimizer algorithm (e.g., Adam optimization algorithm). The loss is also back-propagated through the network, and the gradients are computed. Based on the gradient values, the network's weights are updated.

The process (beginning with the image batch read) is continuously repeated until training samples are still available (until the end of the epoch) at 507.

Then, at 508, the performance metrics are calculated using a validation dataset - which is not explicitly used in training set. This is done in order to check at 509 whether the model has improved. If it is not the case, the early stop counter is incremented at 514 and it is checked at 515 if its value has reached a predefined number of epochs. If so, then the training process is complete at 516, since the model has not improved for many sessions now, so it can be concluded that the network started overfitting to the training data.

If the model has improved, it is stored at 510 for further use and the early stop counter is reset at 511.

As the final step in a session, learning rate scheduling can be applied. The session at which the rate is to be changed are predefined. Once one of the session numbers is reached at 512, the learning rate is set to one associated with this specific session number at 513.
Once the training is complete, the network can be used for inference (i.e., utilizing a trained model for prediction on new data).

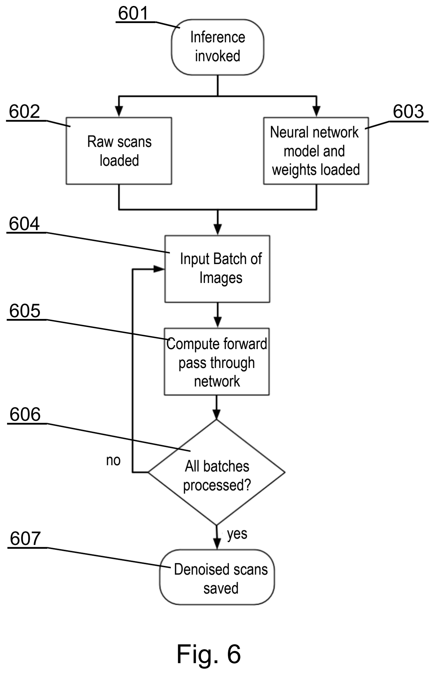

Fig. 6 shows a flowchart of an inference process for the denoising CNN 300.

After inference is invoked at 601, a set of scans (LDCT, not denoised) are loaded at 602 and the denoising CNN 300 and its weights are loaded at 603.

At 604, one batch of images at a time is processed by the inference server. At 605, a forward pass through the denoising CNN 300 is computed.

At 606, if not all batches have been processed, a new batch is added to the processing pipeline until inference has been performed at all input noisy LDCT images.

Finally, at 607, the denoised scans are stored.

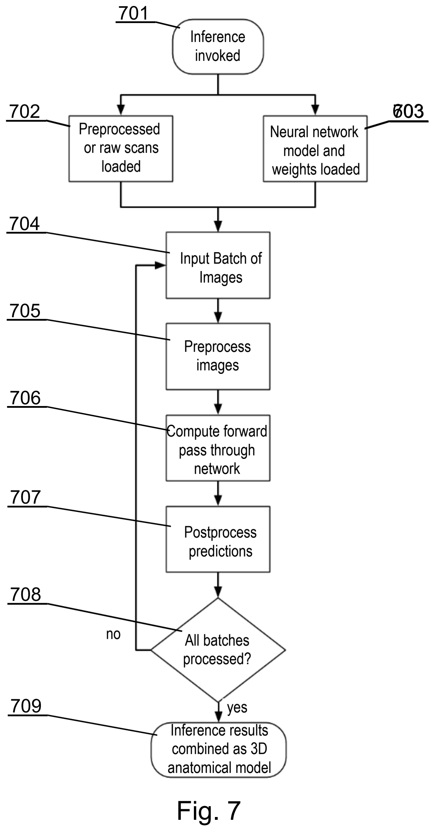

Fig. 7 shows a flowchart of an inference process for the marker detection CNN 400. After inference is invoked at 701, a set of raw scans or denoised images are loaded at 702 and the marker detection CNN 400 and its weights are loaded at 703.

At 704, one batch of images at a time is processed by the inference server.

At 705, the images are preprocessed (e.g., normalized and/or cropped) using the same parameters that were utilized during training, as discussed above. In at least some implementations, inference-time distortions are applied and the average inference result is taken on, for example, 10 distorted copies of each input image. This feature creates inference results that are robust to small variations in brightness, contrast, orientation, etc.

At 706, a forward pass through the marker detection CNN 400 is computed.

At 707, the system may perform post-processing such as thresholding, linear filtering (e.g., Gaussian filtering), or nonlinear filtering, such as median filtering and morphological opening or closing.

At 708, if not all batches have been processed, a new batch is added to the processing pipeline until inference has been performed at all input images.

Finally, at 709, the inference results are saved and images such as shown in Fig. 1E or 1F can be output.

The functionality described herein can be implemented in a computer system. The system may include at least one nontransitory processor-readable storage medium that stores at least one of processor-executable instructions or data and at least one processor communicably coupled to that at least one nontransitory processor-readable storage medium. That at least one processor is configured to perform the steps of the methods presented herein.

### CLAUSES RELATED TO INVENTION(S) DESCRIBED HEREIN

1. A computer-implemented system, comprising:
   - at least one nontransitory processor-readable storage medium that stores at least one of processor-executable instructions or data; and
   - at least one processor communicably coupled to at least one nontransitory processor-readable storage medium, wherein at least one processor:
      - reads (811) the set of images, each comprising an image of a registration grid (181) comprising an array of markers (181B);
      - detects (812) the markers on each of the images by using a marker detection convolutional neural network (CNN);
      - filters (821) the detection results to remove false positives; and
      - determines (831) the 3D location and 3D orientation of the registration grid (181) based on the detected markers (181B).
2. The system according to claim 1, wherein at least one processor further:
   - receives marker detection learning data comprising a plurality of batches of labeled anatomical image sets, each image set comprising image data representative of an anatomical structure, and each image set including at least one marker;
   - trains a fully convolutional neural network model (marker detection CNN) to detect markers on the images using the received marker detection learning data; and
   - stores the trained marker detection CNN model in at least one nontransitory processor-readable storage medium of the machine learning system.
3. The system according to any of previous claims, wherein at least one processor further:
   - receives denoising learning data comprising a plurality of batches of high quality medical imagery and low quality medical imagery; and
   - trains a fully convolutional neural network model (denoising CNN) to denoise an image utilizing the received denoising learning data; and stores the trained denoising CNN model in at least one nontransitory processor-readable storage medium of the machine learning system.
4. The system according to claim 3, wherein at least one processor further operates the trained marker detection CNN to process a set of input anatomical images to detect markers.
5. The system according to claim 4, wherein at least one processor further operates the trained denoising CNN to process a set of input anatomical images to generate a set of output denoised anatomical images.
6. The system according to claim 5, wherein the set of input anatomical images for the trained denoising CNN comprises the low quality anatomical images.
7. The system according to claim 5, wherein the set of input anatomical images for the trained marker detection CNN comprises the set of output denoised anatomical images of the denoising CNN or raw data scan..
8. The system according to any of claims 3-7, wherein the low quality anatomical images are low-dose computed tomography (LDCT) or low-power magnetic resonance images and wherein the high quality anatomical images are high-dose computed tomography (HDCT) or high power magnetic resonance images, respectively.
9. A method for identification of transformation of a predefined object in a set of images from medical image scanner, the method comprising the steps of:
   - reading (811) the set of images, each comprising an image of a registration grid (181) comprising an array of markers (181B);
   - detecting (812) the markers on each of the images by using a marker detection convolutional neural network (CNN);
   - filtering (821) the detection results to remove false positives; and
   - determining (831) the 3D location and 3D orientation of the registration grid (181) based on the detected markers (181B).

## Claims

1. A computer-implemented system, comprising:
at least one non-transitory processor-readable storage medium that stores at least one of processor-executable instructions or data; and
at least one processor communicably coupled to the at least one non-transitory processor-readable storage medium, wherein the at least one processor is configured to:
obtaining a set of two dimensional, 2D, image slices of an intraoperative three-dimensional, 3D, volume, wherein each 2D image slice of the set of 2D image slices comprises an image of an anatomical structure and of a registration grid (181) containing an array of markers (181B) and is accompanied with marker detection results that comprise a binary image with marked pixels that may belong to a marker;;
filter (821) the marker detection results for the set of 2D image slices based on a known size of each marker of the array of markers by (i) processing the whole set of the 2D image slices of the intraoperative 3D volume to group neighboring voxels as blobs, and (ii) removing false positive markers that incorrectly marked blobs by comparing the size of the blobs to the known size of each marker from the array of markers, to obtain filtered marker detection results for the intraoperative 3D volume;
determine, from the filtered marker detection results for the intraoperative 3D volume, a center of each marker from the array of markers; and
determine (831) a center location and 3D orientation of the registration grid (181) with respect to the intraoperative 3D volume by aligning the centers of the array of markers with markers in a reference 3D volume of the registration grid and creating homogeneous transformation matrices that describe the center location and 3D orientation of the registration grid in the intraoperative 3D volume.

2. The system according to claim 1, wherein the at least one processor is further configured to:
receive marker detection learning data comprising a plurality of batches of labeled anatomical image sets, each image set comprising a 2D image slice representative of an anatomical structure, and each image set including at least one marker from the array of markers;
train the marker detection CNN that is based on a fully convolutional neural network model to detect markers on the set of 2D image slices using the received marker detection learning data; and
store the trained marker detection CNN model in at least one non-transitory processor-readable storage medium of the system.

3. The system according to any of previous claims, wherein the at least one processor is further configured to:
receive denoising learning data comprising a plurality of batches of high quality medical 2D image slices of a 3D volume and low quality 2D image slices of the 3D volume; and
train a denoising convolutional neural network, CNN, that is based on a fully convolutional neural network model to denoise a 2D image slice utilizing the received denoising learning data; and
store the trained denoising CNN model in at least one non-transitory processor-readable storage medium of the system.

4. The system according to claim 4, wherein the at least one processor is further configured to operate the trained denoising CNN to process the set of 2D image slices to generate a set of output denoised 2D image slices.

5. The system according to claim 5, wherein the set of 2D image slices for the trained denoising CNN comprises the low quality 2D image slices.

6. The system according to claim 5, wherein the set of 2D image slices for the trained marker detection CNN comprises the set of output denoised 2D image slices of the denoising CNN or raw data scan.

7. The system according to any of claims 3-7, wherein the set of 2D image slices includes low quality 2D image slices that are low-dose computed tomography, LDCT, or low-power magnetic resonance images and high quality 2D image slices that are high-dose computed tomography, HDCT, or high-power magnetic resonance images, respectively.

8. A method for identification of transformation of a predefined object in a set of 2D images from medical image scanner, wherein the set of 2D images is a set of two-dimensional, 2D, image slices of an intraoperative three-dimensional, 3D, volume, wherein each 2D image slice of the set of 2D image slices comprises an image of an anatomical structure and of a registration grid (181) containing an array of markers (181B) and is accompanied with marker detection results that comprise a binary image with marked pixels that may belong to a marker, the method comprising the steps of:
filtering (821) the marker detection results for the set of 2D image slices based on a known size of each marker of the array of markers by (i) processing the whole set of the 2D image slices of the intraoperative 3D volume to group neighboring voxels as blobs, and (ii) removing false positive markers that are incorrectly marked blobs by comparing the size of the blobs to the known size of each marker from the array of markers, to obtain filtered marker detection results for the intraoperative 3D volume;
determining, from the filtered marker detection results for the intraoperative 3D volume, a center of each marker from the array of markers; and
determining (831) a center location and 3D orientation of the registration grid (181) with respect to the intraoperative 3D volume, by aligning the centers of the array of markers with markers in a reference 3D volume of the registration grid and creating homogeneous transformation matrices that describe the center location and 3D orientation of the registration grid in the intraoperative 3D volume.
